# EUROPEAN PATENT APPLICATION

(11) **EP 4 170 663 A1**
(43) Date of publication of application: **26.04.2023**
(21) Application number: 21838637.3
(22) Date of filing: 05.07.2021
(51) Int. Cl.: G16H 10/20, G16H 10/60, G06F 16/242, G06F 16/2453

(54) **APPARATUS, METHOD, AND COMPUTER-READABLE STORAGE MEDIUM FOR SELECTING CLINICAL TRIAL SUBJECT**

(30) Priority: 07.07.2020 KR 20200083340
(71) Applicant: The Asan Foundation, Seoul 05505 (KR); University Of Ulsan Foundation For Industry Cooperation, Ulsan 44610 (KR)
(72) Inventor: LIM, Young-Suk, Seoul 06280 (KR); OH, Ji Seon, Seoul 04773 (KR)
(74) Representative: BCKIP Part mbB
(86) International application number: PCT/KR2021/008507
(87) International publication number: WO 2022/010207

(57) **Abstract**

The present disclosure relates to a method for selecting a clinical trial subject using an apparatus, a plurality of first data corresponding to a plurality of first candidate subjects are extracted from medical data stored in a database by using a first query, a plurality of second candidate subjects are selected from among the plurality of first candidate subjects by using a second query corresponding to the plurality of first data and a plurality of variable conditions, a plurality of third candidate subjects are selected from among the plurality of second candidate subjects based on the displaying of information corresponding to at least a part of second data corresponding to the plurality of second candidate subjects. the clinical trial subject is selected based on the displaying of information corresponding to at least a part of third data corresponding to each of the plurality of third candidate subjects.

## Description

### [Technical Field]

The present disclosure relates to an apparatus, method, non-transitory computer readable recording medium, and computer program for selecting clinical trial subject.

### [Background Art]

Clinical trials are tests conducted on humans to prove the safety and effectiveness of drugs and medical devices. In order to quickly and efficiently conduct the clinical trials, selection of clinical trial subjects is important. Accordingly, in recent years, the importance of technology for using previously accumulated and stored medical data for the selection of subjects for the clinical trial has been brought into light.

Conventionally, a large amount of medical data has been accumulated and stored in a hospital information system of each medical institution, but it is difficult to select necessary medical data in respective hospital information systems of medical institutions in order to use the data to select a clinical trial subject. For example, currently, different hospital information systems are established for each medical institution and each of the systems has medical data in different formats, so it is difficult to collect, integrate, and analyze medical data from various medical institutions. In addition, when performing filtering in a conventional hospital information system in order to analyze medical data, there may be deterioration in accuracy and usability of the medical data depending on which query (condition) is used. In addition, in the case of a conventional technology for searching medical data using a query determined through a direct input by a user, it is difficult to select precise medical data from large-scale data, manual reviews of unspecified numbers may be required, and there may be a possibility of leakage of the medical data.

Meanwhile, in recent years, medical data and search conditions for medical data have become more and more complex, and there is a difficulty in determining a query for medical data search. In addition, when a query includes complex conditions, there may be limitations in development of a user interface, and when medical data is retrieved using such a query including the complex conditions, there may be problems such as slow processing speed and repeated execution in a server. Accordingly, it is required to develop new technologies for screening high-accuracy medical data from a large amount of medical data of medical institutions.

### [Disclosure]

### [Technical problem]

According to an embodiment of the present disclosure, there is provided an apparatus, method, non-transitory computer readable recording medium, and computer program for selecting a clinical trial subject based on a large amount of medical data accumulated and stored in medical institutions.

According to an embodiment of the present disclosure, there is provided a technology to efficiently search for and finally select a subject satisfying complex clinical trial conditions based on medical data stored as large-scale and complex medical data so as to quickly and accurately select clinical trial subjects for a user.

### [Technical Solution]

In accordance with an aspect of the present disclosure, there is provided a method for selecting a clinical trial subject using an apparatus, the method comprising: extracting a plurality of first data corresponding to a plurality of first candidate subjects from medical data stored in a database by using a first query, and storing the extracted first data in a database; selecting a plurality of second candidate subjects from among the plurality of first candidate subjects by using a second query corresponding to the plurality of first data and a plurality of variable conditions; selecting a plurality of third candidate subjects from among the plurality of second candidate subjects based on the displaying of information corresponding to at least a part of second data corresponding to the plurality of second candidate subjects; and selecting the clinical trial subject based on the displaying of information corresponding to at least a part of third data corresponding to each of the plurality of third candidate subjects, wherein the information corresponding to the at least a part of the second data corresponding to the plurality of second candidate subjects includes information classified by preset items and displayed, wherein the selecting of the plurality of third candidate subjects from among the plurality of second candidate subjects comprises: changing a condition corresponding to at least one item among the preset items based on receiving a user input regarding at least a part of the information classified by the preset items and displayed; and selecting the plurality of third candidate subjects based on the changed condition; wherein the information classified by the preset items and displayed includes statistical information for each item of the plurality of second candidate subjects, and wherein the preset items include age, gender, diagnosis, a physician in charge, a medical department, hospital visit history, medication history, or test information.

According to various embodiments, the plurality of variable conditions may include a combination of at least some of a plurality of unit queries, the method further comprises generating the second query based on a combination of the at least some of the plurality of unit queries, each of the plurality of unit queries may correspond to one of preset simple conditional queries.

According to various embodiments, the method further comprises displaying a screen for setting the second query based on the plurality of first data; and based on a user input received in response to the displaying of a screen for setting the second query, changing the at least some of the plurality of unit queries or changing the combination of the at least some of the plurality of unit queries.

According to various embodiments, the displaying of the information corresponding to the at least a part of the second data corresponding to the plurality of second candidate subjects comprises classifying the second data by items corresponding to at least a part of the combined queries among the plurality of unit queries, and displaying at least a part of the second data classified by items.

According to various embodiments, the information corresponding to the at least a part of the third data corresponding to each of the plurality of third candidate subjects may be displayed in sequence.

In accordance with other aspect of the present disclosure, there is provided a non-transitory computer-readable recording medium having a computer program stored therein, wherein the computer program is, when being executed by a processor, configured to perform a method comprising: extracting a plurality of first data corresponding to a plurality of first candidate subjects from entire medical data stored in a database by using a first query, and store the extracted first data in the database; selecting a plurality of second candidate subjects from among the plurality of first candidate subjects by using a second query corresponding to the plurality of first data and a plurality of variable conditions; selecting a plurality of third candidate subjects from among the plurality of second candidate subjects based on the displaying of information corresponding to at least a part of second data corresponding to the plurality of second candidate subjects; and selecting a clinical trial subject based on the displaying of information corresponding to at least a part of third data corresponding to each of the plurality of third candidate subjects, wherein the information corresponding to the at least a part of the second data corresponding to the plurality of second candidate subjects includes information classified by preset items and displayed, wherein the selecting of the plurality of third candidate subjects from among the plurality of second candidate subjects comprises: changing a condition corresponding to at least one item among the preset items based on receiving a user input regarding at least a part of the information classified by the preset items and displayed; and selecting the plurality of third candidate subjects based on the changed condition; wherein the information classified by the preset items and displayed includes statistical information for each item of the plurality of second candidate subjects, and wherein the preset items include age, gender, diagnosis, a physician in charge, a medical department, visit history, medication history, or test information.

In accordance with another aspect of the present disclosure, there is provided an apparatus for selecting a clinical trial subject, the apparatus comprising: a communication interface; a display; a processor electrically connected to the communication interface and the display; and a memory electrically connected to the processor, wherein the processor is, when the memory is executed, configured to: extract a plurality of first data corresponding to a plurality of first candidate subjects from medical data stored in a database by using a first query, and store the extracted first data in a database; select a plurality of second candidate subjects from among the plurality of first candidate subjects by using a second query corresponding to the plurality of first data and a plurality of variable conditions; select a plurality of third candidate subjects from among the plurality of second candidate subjects based on the displaying of information corresponding to at least a part of second data corresponding to the plurality of second candidate subjects; and select the clinical trial subjects based on the displaying of information corresponding to at least a part of third data corresponding to each of the plurality of third candidate subjects, wherein the information corresponding to the at least a part of the second data corresponding to the plurality of second candidate subjects comprises information classified by preset items and displayed, wherein the selecting of the plurality of third candidate subjects from among the plurality of second candidate subjects comprises: changing a condition corresponding to at least one item among the preset items based on receiving a user input regarding at least a part of the information classified by the preset items and displayed; and selecting the plurality of third candidate subjects based on the changed condition; wherein the information classified by the preset items and displayed includes statistical information for each item of the plurality of second candidate subjects, and wherein the preset items include age, gender, diagnosis, a physician in charge, a medical department, hospital visit history, medication history, or test information.

### [Advantageous Effects]

In the apparatus, method, non-transitory computer readable recording medium, and computer program for selecting a clinical trial subject according to an embodiment of the present disclosure, it is possible to reduce the burden of data processing of the apparatus and select a clinical trial subject through a rapid and accurate condition search. For example, it is possible to improve the speed, accuracy, convenience, and efficiency of selection of a clinical trial subject by combining the advantages of various methods having different characteristics. In addition, it is possible to overcome the limitations of a clinical trial subject selecting method based on large-scale medical data of a hospital information system and contribute to improvement of performance of clinical trials.

### [Description of Drawings]

FIG. 1 is a block diagram of an apparatus for selecting a clinical trial subject according to an embodiment of the present disclosure.
FIG. 2 is a flowchart of an operation of the apparatus for selecting a clinical trial subject according to the embodiment of the present disclosure.
FIGS. 3 to 6 are diagrams for explaining an operation of the apparatus for selecting a clinical trial subject according to an embodiment of the present disclosure.
FIGS. 7 and 8 are diagrams illustrating examples of screens displaying information corresponding to at least a part of second data corresponding to a plurality of secondly selected candidate subjects according to an embodiment of the present disclosure.

### [Mode for Disclosure]

The advantages and features of embodiments and methods of accomplishing these will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined by the scope of the appended claims.

In describing the embodiments of the present disclosure, if it is determined that detailed description of related known components or functions unnecessarily obscures the gist of the present disclosure, the detailed description thereof will be omitted. Further, the terminologies to be described below are defined in consideration of functions of the embodiments of the present disclosure and may vary depending on a user's or an operator's intention or practice. Accordingly, the definition thereof may be made on a basis of the content throughout the specification.

Hereinafter, embodiments of the present disclosure will be described in detail with reference to the accompanying drawings.

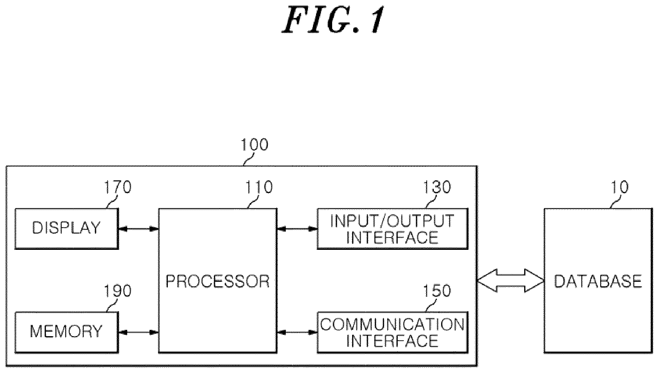

FIG. 1 is a block diagram of an apparatus for selecting a clinical trial subject according to an embodiment of the present disclosure.

Referring to FIG. 1, an apparatus 100 may include a processor 110, an input/output interface 130, a communication interface 150, a display 170, and/or a memory 190.

The processor 110 (also referred to as a controller, a control device, or a control circuit) may control at least one component of the apparatus 100 other than itself (e.g., a hardware component (e.g., the input/output interface 130, the communication interface 150, the display 170, and/or the memory 190) or a software component) connected to the processor 110, and may perform various data processing and operations.

The input/output interface 130 may transfer, for example, a command or data input from a user or an external device to components of the apparatus 100, or may output a command or data received from the components of the apparatus 100 to the user or the external device, for example, a database 10.

The communication interface 150 may establish communication between the apparatus 100 and an external device, for example. For example, the communication interface 150 may be connected to a network through wireless or wired communication to communicate with the external device, for example, the database 10.

The display 170 (or touch screen) is a device for visually providing information to a user of the apparatus 100, and may further include a control circuit for controlling the corresponding device.

The memory 190 may store various types of data used by at least one component of the apparatus 100 (the processor 110, the input/output interface 130, the communication interface 150, and/or the display 170),and for example, a software (e.g., a program) and input data or output data for a command related thereto may be stored. The memory 190 may include a volatile memory or non-volatile memory.

According to one embodiment, the memory 190 may store a program (or application) for selecting a clinical trial subject.

According to one embodiment, by using a first query, the processor 110 may retrieve a plurality of first data corresponding to a plurality of first candidate subjects from entire medical data stored in the database 10 using the input/output interface 130 or the communication interface 150. By using a second query corresponding to the plurality of first data and a plurality of variable conditions, the processor 110 may select a plurality of second candidate subjects from among the plurality of first candidate subjects. The processor 110 may control the display 170 to select a plurality of third candidate subjects from among the plurality of second candidate subjects based on display information corresponding to at least a part of the second data corresponding to the plurality of second candidate subjects. The processor 110 may control the display 170 to display information corresponding to at least a part of third data corresponding to each of the plurality of third candidate subjects to select the clinical trial subjects.

According to one embodiment, the database 10 may be a database having medical data stored therein, and may also be referred to as a clinical data warehouse. In the database 10, medical data (also referred to as data) of hundreds to tens of millions of patients may be stored in various forms such as tables or the like.

For example, the processor 110 may generate the second query based on a combination of at least some of a plurality of unit queries. Each of the plurality of unit queries may correspond to one of preset simple conditional queries.

For example, the processor 110 may control the display 170 to display a screen for setting the second query based on the plurality of first data. In response to displaying the screen for setting the second query, the processor 110 may change at least a part of the plurality of unit queries or may change a combination method of the at least a part based on a user input received through the input/output interface 130.

For example, presentation of information corresponding to at least a part of the second data corresponding to the plurality of second candidate subjects may include classifying the second data by items corresponding to at least a part of the combined queries among the plurality of unit queries and presenting at least a part of the second data classified by the items through the display 170. For example, the items may include age, gender, diagnosis, physician in charge, medical department, visit history, medication history, or test information.

For example, the information corresponding to at least a part of the second data corresponding to the plurality of second candidate subjects may include information classified by preset items and displayed. Based on receiving a user input for at least a part of the displayed information, the processor 110 may change a condition corresponding to at least one of the items. The processor 110 may select the plurality of third candidate subjects based on the changed condition.

For example, information corresponding to at least a part of the third data corresponding to each of the plurality of third candidate subjects may be displayed in sequence (or a time-series manner).

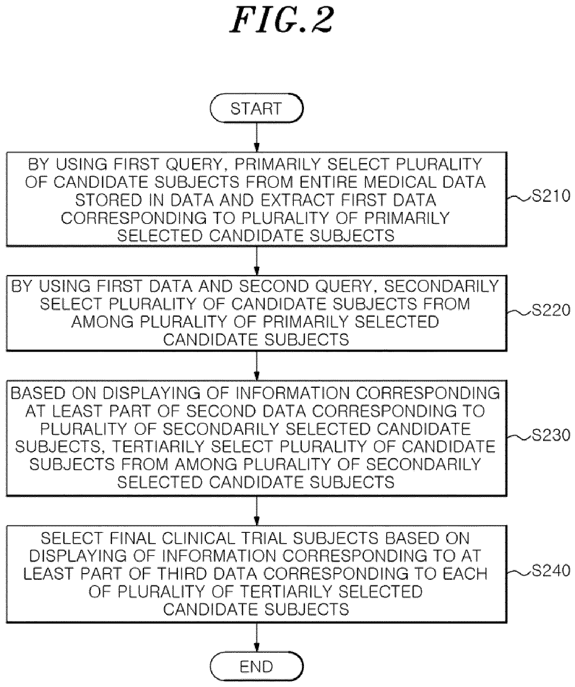

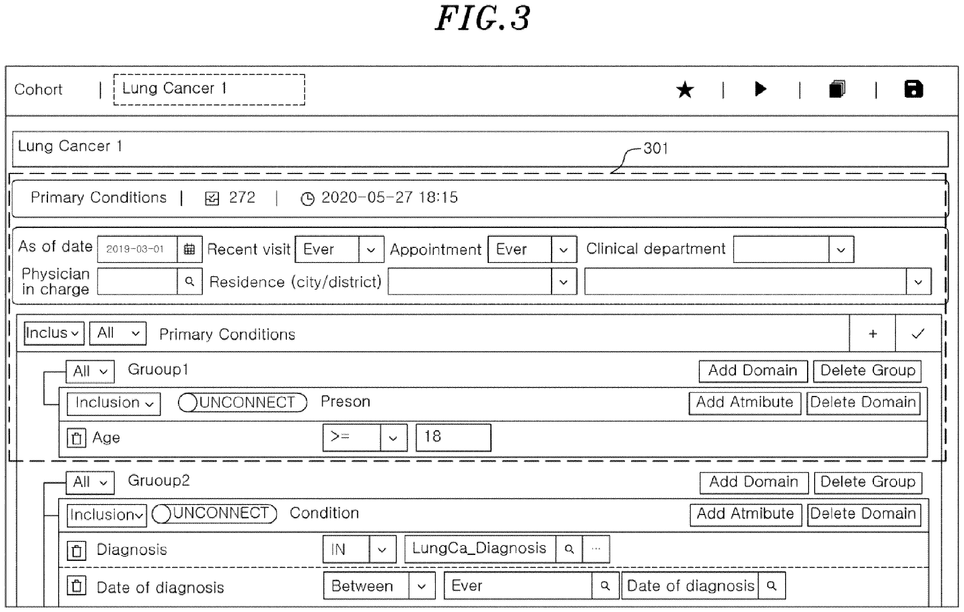

FIG. 2 is a flowchart of an operation of the apparatus 100 (or the processor 110 of the apparatus 100) for selecting a clinical trial subject according to an embodiment of the present disclosure. FIGS. 3 to 6 are diagrams illustrating a display screen of a program for explaining operations of the apparatus 100 (or the processor 110 of the apparatus 100) to select clinical trial subjects according to an embodiment of the present disclosure.

Referring to FIGS. 2 to 6, the apparatus 100 may selects clinical trial subjects (or also referred to as subjects) through multi-step search in medical data stored in a hospital information system, for example, a database (e.g., the database 10).

In operation S210, the apparatus 100 may primarily select a plurality of candidate subjects (also referred to as first candidate subjects) from entire medical data stored in the database 10 using a first query, and extract first data corresponding to a plurality of primarily selected candidate subjects.

According to one embodiment, a memory (e.g., the memory 170) of the apparatus 100 may store a program (or also referred to as an application) for selecting a clinical trial subject, and the apparatus 100 may execute the program based on a user input. The apparatus 100 may display a screen for setting the first query based on the execution of the program.

For example, on the screen for setting the first query, the apparatus 100 may select a plurality of first candidate subjects corresponding to various conditions necessary for a clinical trial through access to a database based on a user input, and may download and acquire data corresponding to the plurality of primary candidate subjects from the database. Referring to FIG. 3, the screen for setting the first query may include first items for setting a query corresponding to an essential invariant condition such as a diagnosis name. For example, the first items may include items to input or select a clinical trial candidate subject's age, gender, clinical department, hospital visit history, diagnosis name, medication history, and/or condition to be mandatorily excluded in a clinical trial, etc., and a condition (also called information) corresponding to each of the items may be set as a query based on a user input. For example, conditions corresponding to the items may be set such that the condition corresponding to age is 60 or older and the condition corresponding to gender is female.

In another example, a first query corresponding to each type of clinical trial may be pre-stored in the apparatus 100, and a screen for setting the pre-stored first query may include items to input or select types of clinical trials. When a type of the clinical trial is input or selected based on a user input, the apparatus 100 may identify a first query that is previously set and stored to correspond to the clinical trial. The apparatus 100 may primarily select a plurality of candidate subjects through access to the database based on the identified first query, and acquire data corresponding to the plurality of candidate subjects primarily selected. In addition, the screen for setting the first query may further include one or more items that can be additionally set as the first query based on user input, in addition to the items to input or select the types of clinical trials.

According to one embodiment, the first query may include one condition or a plurality of conditions.

For example, the first query is a simple query and may include a query for extracting first data by primarily selecting clinical trial candidate subjects from all data stored in the database 10.

For example, the first query may include first items and conditions (also referred to as information) corresponding to each of the first items. The first items may include age, diagnosis, clinical department, hospital visit history, and/or condition to be excluded. Conditions corresponding to each of the first items may be such that the subject is 20 years or older, the diagnosis includes a non-small cell lung cancer disease group, the clinical departments include oncology and respiratory departments, the hospital visit history includes the last year, and the condition to be excluded is death.

According to one embodiment, the first query is configured as a simple query, so that a time for the apparatus 100 to access the database to search for data can be minimized. In addition, in the case of additionally selecting data based on a complex query (also referred to as a second query) after selecting a plurality of primary candidate subjects based on a first query, it is possible to perform multi-viewpoint search and repetitive simulation based on a plurality of data selected based on the first query, which can be referred to as the minimum data set.

According to one embodiment, the apparatus 100 may set a first query 301 based on a user input, and, based on the first query 301, the apparatus 100 may primarily select some patients corresponding to the first query 301 from among patients stored in the database 10 as clinical trial candidate subjects. The number of candidate subjects primarily selected may be set based on a user input, or may be preset and stored. For example, the apparatus 100 may select hundreds to thousands of candidate subjects from the database 10 and acquire information tables related to the candidate subjects, for example, about 10 information tables. The information tables are merely examples and may be changed in various forms.

According to one embodiment, the apparatus 100 may store first data (or also referred to as a data set) corresponding to the plurality of primarily selected candidate subjects in the memory of the apparatus 100, a separate database, or temporary storage.

For example, the first data may include study numbers randomly generated for the plurality of primary candidate subjects, at least some basic information (gender, age, and/or place of residence, etc.) related to each of the plurality of primary candidate subjects, and/or at least some medical information (medical history, treatment history, medical test results, etc.) related to each of the plurality of candidate subjects.

In operation S220, the apparatus 100 may secondarily select a plurality of candidate subjects (also referred to as second candidate subjects) from among the plurality of initially selected candidate subjects by using the first data and the second query.

According to one embodiment, without additional access to the database in which all medical data is stored, the apparatus 100 may quickly secondarily select candidate subjects from the first data corresponding to the plurality of primarily selected candidate subjects using a second query, thereby reducing the candidate subjects.

According to one embodiment, the apparatus 100 may display a screen for setting the second query after primarily selecting the plurality of candidate subjects. For example, the screen for setting the second query may include second items for setting the second query. The second items may be determined based on a type of clinical trial (examination name) and/or a user input. The second items may include medication history, surgical history, and/or condition to be excluded. Each of the second items may correspond to a unit query.

According to one embodiment, the apparatus 100 may generate (or set) the second query as a combination of a plurality of unit queries based on a user input or a predetermined condition.

For example, the apparatus 100 may selectively determine items of the unit queries based on a user input, and set conditions (also referred to as information) corresponding to the items of the unit queries as a second query.

In another example, the apparatus 100 may generates the plurality of unit queries into a second query, which is a complex conditional query, by using various optional combinations (e.g., union, intersection, and/or difference) of the plurality of unit queries according to preset condition.

According to one embodiment, the second query may include a plurality of unit queries based on simple conditional queries (or referred to as simple conditions) for complex conditions required for a clinical trial.

For example, the second query may include second items and conditions (also referred to as information) corresponding to each of the second items. The second items may include medication history, surgical history, oncogenic mutation, and/or conditions to be excluded. The conditions respectively corresponding to the second items may be, for example, the presence or absence of specific targeted anticancer therapy within the last 6 months in terms of medication history, the presence or absence of pulmonary resection in terms of surgical history, the presence or absence of EGFR in terms of cancer genetic mutation, and severe infection and severe liver function decline within the last 3 months in terms of conditions to be excluded.

For example, in the second query, an item and/or a condition (also referred to as information) corresponding to the item may be changed according to a user input, and accordingly, the second query may be referred to as a query with variable conditions.

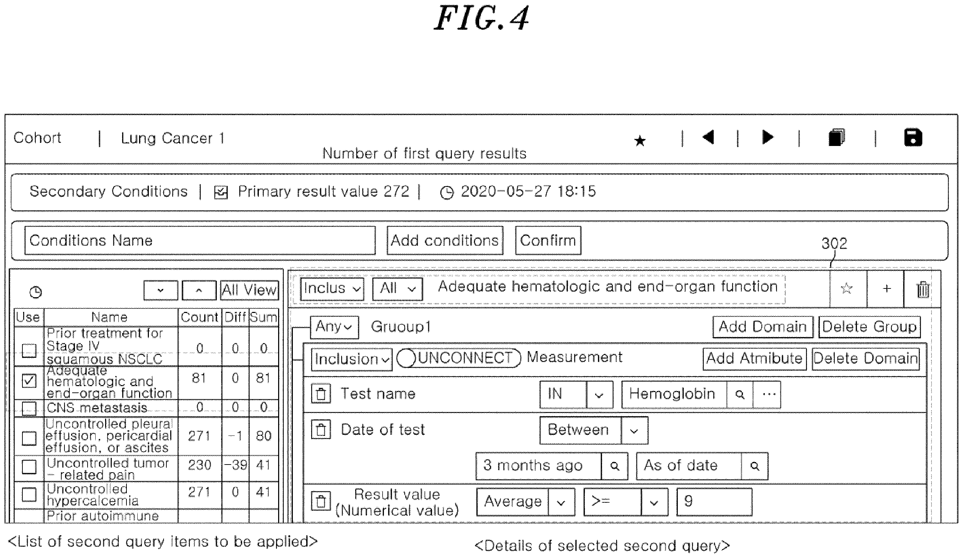

Referring to FIG. 4, the apparatus 100 may secondarily select a plurality of candidate subjects based on a second query 302. Data corresponding to the candidate subjects secondly selected based on the plurality of unit queries may be simplified by being limited to a list of subjects satisfying individual conditions to be described later.

For example, the number of secondly selected candidate subjects may be set based on a user input, or may be previously set and stored. For example, the apparatus 100 may select tens of candidate subjects.

For example, as shown on the left side of FIG. 4, the apparatus 100 may include or exclude an item in or from the second query, by marking an item to be used as the second query based on a user's input. In addition, the apparatus 100 may allow a user to check the number of persons selected as second candidate subjects or candidate subject information according to any of various optional combinations of the second query, while checking an item to be included in the second query by referring to information such as a change in the number of candidate subjects. In this way, a simulation may be performed in such a way that the number of candidate subjects selected based on whether or not corresponding query items are marked is immediately applied and displayed.

According to one embodiment, the apparatus 100 may secondarily select a plurality of candidate subjects (also referred to as a set of candidate subjects selected by unit), which is a comprehensive result based on a secondary query that is a combination of the plurality of unit queries) thereby realizing the effect of complex queries in real time.

In operation S230, the apparatus 100 may tertiarily select a plurality of candidate subjects from among the secondly selected candidate subjects based on the displaying of information corresponding to at least a part of second data corresponding to the plurality of secondarily selected candidate subjects.

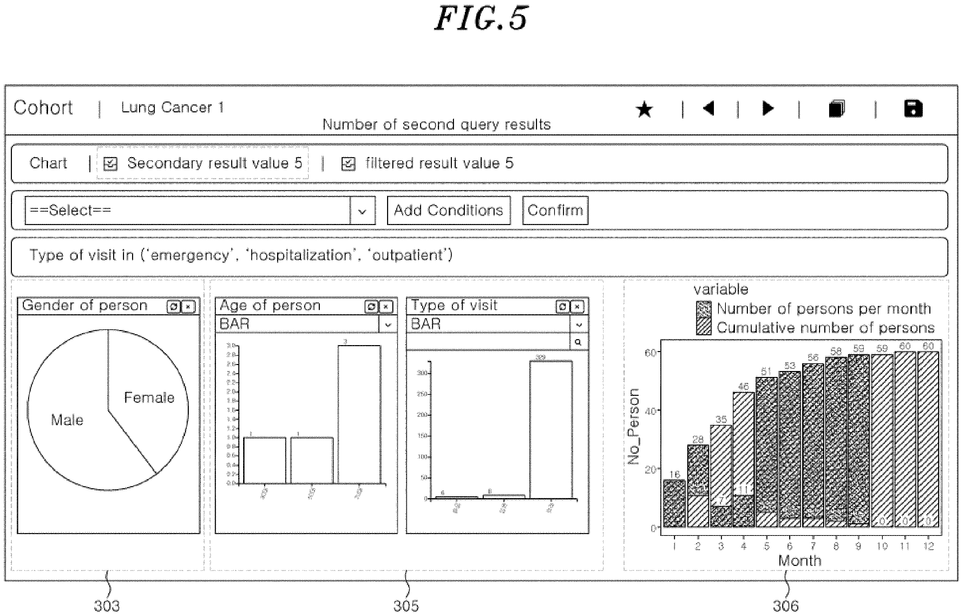

According to one embodiment, as shown in FIG. 5, based on the secondarily selected plurality of candidate subjects, the apparatus 100 may visually display, on a screen, second data corresponding to the plurality of candidate subjects (the gender ratio of the plurality of candidate subjects, a chart 303 showing the number of persons by gender, which is one of the second query items, a chart 305 showing the number of persons by age or number of visits, which is one of the second query items, and a chart 306 showing the number of persons by number of visits per month or number of cumulative number visits, which is one of the second query items).

For example, the apparatus 100 may classify the second data corresponding to the plurality of secondly selected candidate subjects by items, and may visually display on the screen some or all information corresponding to the second data classified by items.

For example, the items may respectively correspond to the unit queries, and the items may include demographic information (age, gender, etc.), diagnosis name, clinical department, visit history, medication history, and/or various test information.

For example, a visual display format may be preset for each item, and the apparatus 100 may display information corresponding to the second data classified by items on the screen in the corresponding visual display format.

In another example, a visual display format corresponding to each item may be selected or set based on a user input, and the apparatus 100 may display on the screen the second data classified by items in the visual display format selected based on the user input.

According to one embodiment, the apparatus 100 may classify second data by items and visually displays the second data on the screen so that a user can visually check a result of the secondary selection, and at the same time, based on a user input, the apparatus 100 may additionally provide a query corresponding to a selection condition that is difficult to provide in the previous steps, or may partially change a corresponding condition for each item. The apparatus 100 may perform an operation of selecting candidate subjects based on the additionally provided query or the query for partially changing a condition corresponding to each item, thereby reducing the number of candidate subjects.

In general, due to the characteristics and limitations of clinical data required for clinical trials, queries for collecting complex information are required, and in order to select more accurate data, the apparatus 100 may repeatedly perform a simulation to select candidate subjects secondarily. For example, the user may repeatedly reset the second query by repeatedly applying a user input, and whenever the second query is set, the apparatus 100 may perform a simulation to select a plurality of candidate subjects, that is, an operation of selecting a plurality of candidate subjects.

For example, while classifying the second data by item and visually displaying the second data on the screen, the apparatus 100 may display a user interface (UI) for changing a condition corresponding to each item. Accordingly, the user may be able to directly check information for each item of a selected candidate subject, and based on a specific review of the information and a user input to the user interface for changing the condition, the apparatus 100 may enable simulation according to a condition of an added item or a change in condition of each item. For example, based on a user input to a user interface for changing the condition, the apparatus 100 may add some items or change conditions corresponding to each of the items, and based on the addition of some items or the change in the conditions the apparatus may tertiarily select a plurality of candidate subject, thereby reducing the number of candidate subjects.

For example, referring to FIG. 5, the apparatus 100 may visually display at least one item of information corresponding to the plurality of candidate subjects (e.g., gender, age (Person), visited clinical department, classification of visit (Visit), diagnosis department, diagnosis code (Condition)), prescription department, prescription code, prescription date (Drug), a result value (Measurement), and examination code (Procedure)) in the form of a chart or graph.

Referring to FIG. 5, the apparatus 100 may select a gender ratio and a classification of visit from information corresponding to the plurality of candidate subjects, and, as shown in FIG. 5, the apparatus 100 may display the chart 303 showing the ratio of gender (female and male), and the chart 305 showing the number of candidate subjects corresponding to outpatient, comprehensive medical examination, and hospitalization according to the number of persons and type of visit of the plurality of candidate subjects belonging to each age group.

For example, as shown in FIG. 3, the apparatus 100 may provide a user interface (UI) 301 for changing conditions, change some of conditions corresponding to respective items based on a user input through the user interface 301, and tertiarily select a plurality of candidate subjects based on the change of some of the conditions.

For example, in a case where, in the secondary screening operation, it is assumed that a first condition is an age of 20 to 80 years, a second condition is a hemoglobin level of 9 or less, a third condition is an absolute neutrophil count, and a fourth condition is visit to the hospital, the apparatus 100 may classify and visualize the extracted data according to items (or also referred to as categories) such as age, examination, and visit, and may provide the visualized data to the user. For example, the apparatus 100 may visualize and provide data (result value) of hemoglobin level and absolute neutrophil count in detail in the item of examination, and may visualize and provide the item of visit so as to confirm whether the corresponding examination has been conducted in the form of outpatient, hospitalization, or emergency.

For example, when the user wishes to consider a group of young patients who are easy to visit a hospital in the first to fourth conditions, the user may control the apparatus 100 to change the first condition through the user interface 301 provided by the apparatus 100 to change conditions. As the first condition is changed, the apparatus 100 may re-simulate the operation of selecting candidate subjects, that is, perform the above-described secondary screening process again. Accordingly, result values for the second condition, the third condition, and the fourth condition may also be changed, and the apparatus 100 may visually provide the changed result values so that the user can check the result values. Through this process, the user is able to review the selection results in detail while changing conditions.

Referring to FIG. 5, the apparatus 100 may select a scheduled visit date from information corresponding to the plurality of candidate subjects, and, as shown in FIG. 5, the apparatus 100 may display a bar graph 306 showing the number of candidate subjects scheduled to visit each month (No_Person) and the cumulative number of candidate subjects scheduled to visit each month.

In general, in a clinical trial, it is important to confirm in advance whether the number of registered people can be secured within a given period. Accordingly, in the embodiment of the present disclosure, as shown in the bar graph 306 of FIG. 5, the apparatus 100 may simulate and visually display the maximum number of people available to be registered in advance according to the scheduled visit dates of reserved patients, and may adjust priorities along with conditions (or various variables) corresponding to each of the above items to derive a selection result for selecting candidate subjects most suitable for the characteristics of the clinical trial.

For example, according to an embodiment of the present disclosure, when a user wishes to select only subjects available to be registered in a clinical trial within 3 months in order to successfully conduct a clinical trial, the chart 305 showing scheduled visit information (emergency, hospitalized, outpatient) of candidate subjects and the bar graph 306 showing the cumulative number of patients scheduled to visit the hospital (potential registration candidates) for each month may be used, as shown in FIG. 5. For example, while the graph 306 in FIG. 5 is displayed, when a user input to select bars corresponding to January, February, and March is received and a user input to select a bar corresponding to outpatient in the chart 305 is received, the apparatus may re-select only candidate subjects scheduled to visit an outpatient clinic in January, February, and March from among the candidate subjects selected through the secondary screening process. Such a change in the candidate list may also affect the results according to conditions for other items, and thus, the apparatus 100 may perform a simulation to select candidate subjects so that the user can check the change in the result in real time.

For example, when a condition corresponding to each item in the secondary screening step is an albumin blood test value of 2.0 or higher, there may be several additional considerations for the corresponding condition. For example, a person may have an albumin blood test level of 2.0 or higher at least once within a period of time, or an average (or minimum or maximum value) of an albumin blood test level of 2.0 or higher. In the above-described first and second selection processes, it may be technically difficult to sufficiently reflect these various considerations to fit the intention, and the user may fail to recognize a difference between the various considerations, resulting in a different selection result than intended. Accordingly, as in the embodiment of the present disclosure, by allowing the user to actually visually see the result of the second selection, it is possible to check an error one more time and clearly add, delete, or change a condition.

For example, in a case where it is necessary to select 10 final candidate subjects, if 50 candidate subjects are selected as a result of selection according to an albumin blood test level of 2.0 or higher, which is one of various conditions, in the secondary selection process, priority may be given to 10 patients with relatively higher albumin blood test levels that reflect health status so that the 10 patients can be judged more suitable as clinical trial subjects. To this end, in order to determine a numerical condition of the albumin blood test, it was conventionally necessary to repeat a process of raising a numerical value step by step, check a result, and adjust the numerical value again until reaching to the appropriate number of candidates. However, in the tertiary selection process through visual distribution according to an embodiment of the present disclosure, the user may be able to check the number of people in each numerical section and may immediately check a result of condition change through a very simple manipulation, and thus, an embodiment of the present disclosure may be useful for simulation considering several items other than the aforementioned albumin blood test condition.

For example, a condition of the first and/or second screening process is an adult aged 20 years or older, and when it is searched for by 20 years or older in a preceding stage, a patient aged 90 years or older, which is generally an age inappropriate for clinical trials, may also be selected as a candidate subject. This problem may similarly occur for other selection conditions, and in many cases, such a problem may not be recognized before visualization of data, so the number of candidate subjects may be determined including an imaginary number in the first and second screening processes. However, according to an embodiment of the present disclosure, the apparatus 100 may reduce and select candidate subjects by visually referring to a distribution of candidate subjects and a user input. In addition, by visually checking the distribution of candidate subjects, the user may be able to check outliers, and accordingly, it is possible to estimate and exclude outliers of the data itself rather than the actual result values of the apparatus 100.

In operation S240, the apparatus 100 may select final clinical trial subjects based on display of third information corresponding to each of the plurality of tertiarily selected candidate subjects.

According to one embodiment, the apparatus 100 may visualize and provide the plurality of tertiarily selected candidate subjects, which are a minimum number of candidate subjects selected in the above-described operations.

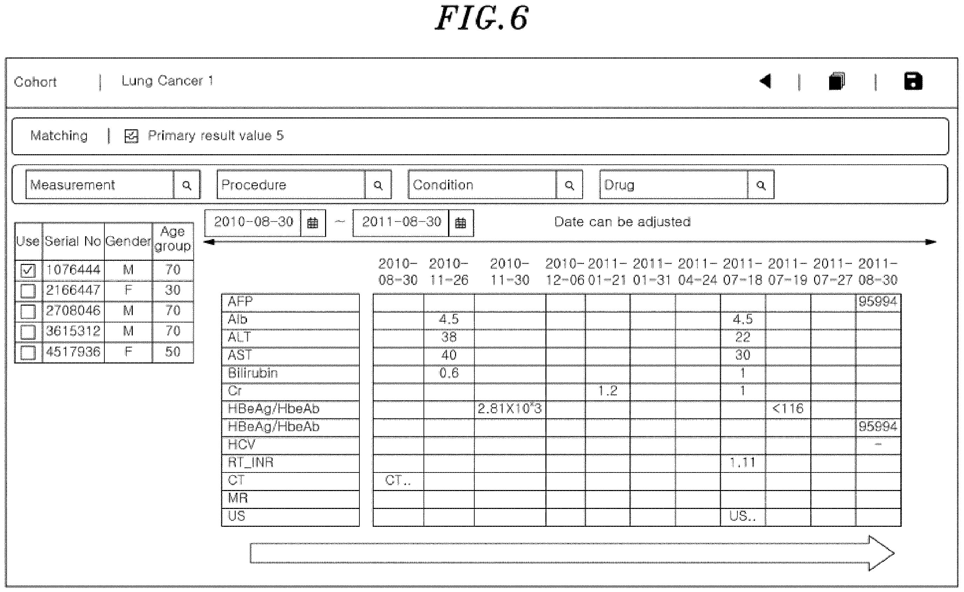

For example, referring to FIG. 6, in the tertiary selection process, when visualization and additional selection are completed and a user input is received (e.g., when a user presses a button of storage or next step), the apparatus 100 may provide a study numbers of a plurality of candidate subjects selected in the third process, select an individual study numbers for each candidate subject one by one based on a user input, and visually provide information (e.g., clinical information) on the selected candidate subject in the form of a table in sequence (or a time-series manner).

Referring to FIG. 6, the apparatus 100 may display a part of information corresponding to the plurality of tertiarily selected candidate subjects in a table form as shown in FIG. 6. A part of the information corresponding to each of the plurality of candidate subjects may include a study number, gender, age group, and the like.

According to one embodiment, the apparatus 100 may visualize and provide third data corresponding to each of the plurality of tertiarily selected candidate subjects, which are a minimum number of candidate subjects selected in the above-described operations, in sequence (or a time-series manner). For example, the apparatus 100 may display visual information including item information for each of the plurality of tertiarily selected candidate subjects on the screen. The apparatus 100 may visualize and provide third data corresponding to a candidate subject corresponding to visual information, which is selected based on a user input from among visual information indicating each of a plurality of candidate subjects displayed, on the screen in sequence (or a time-series manner).

For example, the apparatus 100 time-sequentially summarizes clinical information, which is information corresponding to the third data for each of a plurality of tertiarily selected candidate subjects, and visually provides the clinical information in the form of a flow sheet. Accordingly, the user may be able to grasp the context of a clinical progress of each of the plurality of tertiarily selected candidate subjects in detail, without additional identification information or chart review.

Referring to FIG. 6, based on a user input, the apparatus 100 display clinical information, which is information corresponding to a specific candidate subject among a plurality of tertiarily selected candidate subjects, for example, a candidate subject with serial number 1076444, through time-sequential visualization. For example, as shown in FIG. 6, the apparatus 100 may display the information, corresponding to the candidate subject with serial number 1076444, classified by date in a table form. In FIG. 6, the horizontal axis may be time (date) and the vertical axis may be composed of various clinical items (diagnosis, medication, test, procedure, behavior, etc.), and each cell where the horizontal and vertical lines intersect may provide a result value of a corresponding item at a corresponding time. The items may be defined as an item of interest in advance or may be added and/or excluded as necessary. In addition, the result value may be defined in advance to show the result value as it is or to show a clinically useful representative value (or summary value). Through such visualization of information of each individual candidate subject, the user may be able to easily and quickly grasp the overall clinical course of one candidate subject in depth. In addition, through a final review of whether or not a subject is suitable to be selected as a candidate subject, the user may consider whether or not to finally include the corresponding subject who is technically difficult to select in the preceding processes or who has not been selected for various reasons.

For example, referring to FIG. 5, while a screen as shown in FIG. 5 is displayed, the apparatus 100 may select subjects capable of participating in a clinical trial by comprehensively reviewing the numerical values of various clinical items (diagnosis, medication, examination, procedure, behavior, etc.), and when a user input to select a candidate subject with the serial number 1076444 is received, the apparatus 100 may display a screen as shown in FIG. 6.

According to one embodiment, based on a user input regarding third data corresponding to each of the plurality of candidate subjects provided through visualization, the apparatus 100 may select at least one candidate subject from among the plurality of candidate subjects as a clinical trial subject. For example, through user reviews for each of the individual candidate subjects, the apparatus 100 may determine whether to include or exclude each of the individual candidate subjects based on a user input, applied through a user interface, to determine a final selected list of candidate subjects.

Meanwhile, in addition to the above-described embodiment, the apparatus 100 may perform standardization of entire medical data before the operation S210. For example, when the entire medical data are made in different formats, the apparatus 100 may standardize the entire medical data by matching the entire medical data to predetermined standard terms and/or codes. Since the standardization is a prior art, a detailed description thereof will be omitted.

In addition, the chart 303 showing the ratio of gender (Female and Male) and the chart 305 showing the number of candidate subjects corresponding to outpatient, comprehensive medical examination, and hospitalization according to a type of visit are merely examples, and may be varied according to various condition values reflected in the first selection and/or the second selection process. For example, the apparatus 100 may control the display 170 to visualize and display information by classifying the information by demographics, diagnosis name, clinical department, type of visit, and/or medication history, which are major categories of classification.

In addition, in the above-described embodiment, displaying information corresponding to at least a part of the second data corresponding to the plurality of secondarily selected candidate subjects has been described with the examples of the charts 303 and 305 and the graph 306 of FIG. 5, but the displaying of the information may be changed in various ways in various embodiments. For example, the chart 305 of FIG. 5 is a diagram showing a screen displaying information corresponding to at least a part of second data corresponding to a plurality of secondarily selected candidate subjects according to an embodiment of the present disclosure.

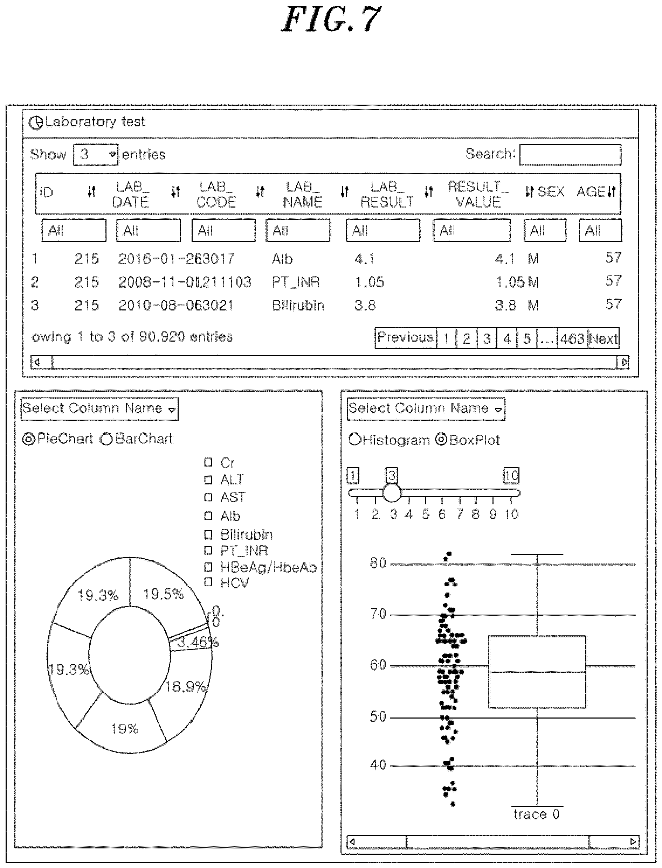

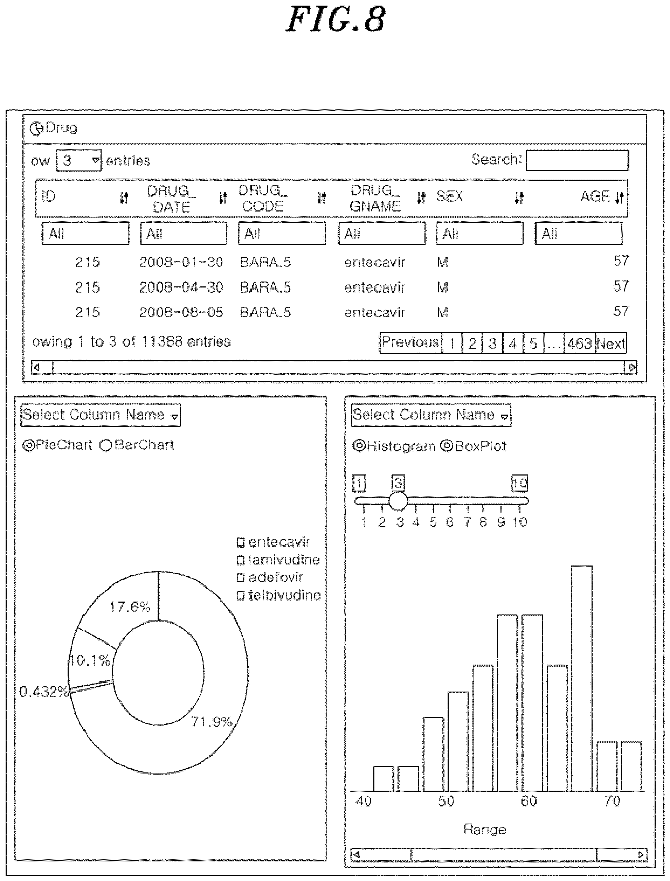

Referring to FIG. 6, entire data may be listed in the form of predefined items, for example, a test result (Measurement), a behavior test (Procedure), a diagnosis (Condition), and a prescription (Drug). In addition, by using a filter function based on user input, for example, as to whether a specific word is included in the case of text data, whether one or multiple categories are included in the case of categorical data, and whether to widen or narrow a range of numbers in the case of numbers, it is possible to adjust a range of data. In addition, a result of the determined data may be reflected in a real-time graph and provided to the user, and the filter may be adjusted again after the user confirms the result. For example, referring to FIG. 7, the user has selected a command All for each item in order to apply a filter function, and based on the user's selection, the apparatus 100 may display a percentage of the number of candidate subjects corresponding to each name in a chart. In addition, the apparatus 100 may display a bar graph for each age group of the candidate subjects. For example, referring to FIG. 8, the user has selected a command All for each item in order to apply a filter function, and based on the user's selection, the apparatus 100 may display a percentage of the number of candidate subjects corresponding to each experiment name in a chart. In addition, the apparatus 100 may display a distribution of candidate subjects by age in a box plot. The above-described filtering method may be inserted inside a table as shown in FIGS. 7 and 8, but a filter may be inserted in a graph to adjust a data range or a specific section or item (also called a category) may be selected on a graph to select a data range.

Various embodiments of the present document may be implemented by software (e.g., a program) including commands stored in a machine-readable storage media (e.g., the memory 190 (internal memory or external memory)) readable by machine (e.g., computer). A machine is a machine capable of calling a stored command from a storage medium and operating in accordance with the called command, and may include an electronic apparatus (e.g., an electronic apparatus 100) according to the disclosed embodiments. When the command is executed by the processor (e.g., the processor 110), the processor may perform a function corresponding to the command directly or by using other components under the control of the processor. A command may include code generated or executed by a compiler or interpreter. The machine-readable storage medium may be provided in the form of a non-transitory storage medium. Here, "non-temporary" only means that the storage medium does not contain a signal and is tangible, but does not mean that data is stored semi-permanently or temporarily in the storage medium.

According to one embodiment, the method according to various embodiments disclosed in this document may be included and provided in a computer program product.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

## Claims

1. A method for selecting a clinical trial subject using an apparatus, the method comprising:
extracting a plurality of first data corresponding to a plurality of first candidate subjects from medical data stored in a database by using a first query, and storing the extracted first data in a database;
selecting a plurality of second candidate subjects from among the plurality of first candidate subjects by using a second query corresponding to the plurality of first data and a plurality of variable conditions;
selecting a plurality of third candidate subjects from among the plurality of second candidate subjects based on the displaying of information corresponding to at least a part of second data corresponding to the plurality of second candidate subjects; and
selecting the clinical trial subject based on the displaying of information corresponding to at least a part of third data corresponding to each of the plurality of third candidate subjects,
wherein the information corresponding to the at least a part of the second data corresponding to the plurality of second candidate subjects includes information classified by preset items and displayed,
wherein the selecting of the plurality of third candidate subjects from among the plurality of second candidate subjects comprises:
changing a condition corresponding to at least one item among the preset items based on receiving a user input regarding at least a part of the information classified by the preset items and displayed; and
selecting the plurality of third candidate subjects based on the changed condition;
wherein the information classified by the preset items and displayed includes statistical information for each item of the plurality of second candidate subjects, and
wherein the preset items include age, gender, diagnosis, a physician in charge, a medical department, hospital visit history, medication history, or test information.

2. The method of claim 1,
wherein the plurality of variable conditions include a combination of at least some of a plurality of unit queries,
wherein the method further comprises generating the second query based on a combination of the at least some of the plurality of unit queries,
wherein each of the plurality of unit queries corresponds to one of preset simple conditional queries.

3. The method of claim 2, further comprising:
displaying a screen for setting the second query based on the plurality of first data; and
based on a user input received in response to the displaying of a screen for setting the second query, changing the at least some of the plurality of unit queries or changing the combination of the at least some of the plurality of unit queries.

4. The method of claim 2, wherein the displaying of the information corresponding to the at least a part of the second data corresponding to the plurality of second candidate subjects comprises
classifying the second data by items corresponding to at least a part of the combined queries among the plurality of unit queries, and displaying at least a part of the second data classified by items.

5. The method of claim 1, wherein the information corresponding to the at least a part of the third data corresponding to each of the plurality of third candidate subjects is displayed in sequence.

6. A non-transitory computer-readable recording medium having a computer program stored therein,
wherein the computer program is, when being executed by a processor, configured to perform a method comprising:
extracting a plurality of first data corresponding to a plurality of first candidate subjects from entire medical data stored in a database by using a first query, and store the extracted first data in the database;
selecting a plurality of second candidate subjects from among the plurality of first candidate subjects by using a second query corresponding to the plurality of first data and a plurality of variable conditions;
selecting a plurality of third candidate subjects from among the plurality of second candidate subjects based on the displaying of information corresponding to at least a part of second data corresponding to the plurality of second candidate subjects; and
selecting a clinical trial subject based on the displaying of information corresponding to at least a part of third data corresponding to each of the plurality of third candidate subjects,
wherein the information corresponding to the at least a part of the second data corresponding to the plurality of second candidate subjects includes information classified by preset items and displayed,
wherein the selecting of the plurality of third candidate subjects from among the plurality of second candidate subjects comprises:
changing a condition corresponding to at least one item among the preset items based on receiving a user input regarding at least a part of the information classified by the preset items and displayed; and
selecting the plurality of third candidate subjects based on the changed condition;
wherein the information classified by the preset items and displayed includes statistical information for each item of the plurality of second candidate subjects, and
wherein the preset items include age, gender, diagnosis, a physician in charge, a medical department, visit history, medication history, or test information.

7. The non-transitory computer-readable recording medium of claim 6,
wherein the plurality of variable conditions include a combination of at least some of a plurality of unit queries,
wherein the method further comprises generating the second query based on a combination of the at least some of the plurality of unit queries,
wherein each of the plurality of unit queries corresponds to one of preset simple conditional queries.

8. The non-transitory computer-readable recording medium of claim 7,
wherein the method further comprises:
displaying a screen for setting the second query based on the plurality of first data; and
based on a user input received in response to the displaying of a screen for setting the second query, changing the at least some of the plurality of unit queries or changing the combination of the at least some of the plurality of unit queries.

9. The non-transitory computer-readable recording medium of claim 7,
wherein the displaying of the information corresponding to the at least a part of the second data corresponding to the plurality of second candidate subjects comprises classifying the second data by items corresponding to at least a part of the combined queries among the plurality of unit queries, and displaying at least a part of the second data classified by items.

10. The non-transitory computer-readable recording medium of claim 6, wherein the information corresponding to the at least a part of the third data corresponding to each of the plurality of third candidate subjects is displayed in sequence.

11. An apparatus for selecting a clinical trial subject, the apparatus comprising:
a communication interface;
a display;
a processor electrically connected to the communication interface and the display; and
a memory electrically connected to the processor,
wherein the processor is, when the memory is executed, configured to:
extract a plurality of first data corresponding to a plurality of first candidate subjects from medical data stored in a database by using a first query, and store the extracted first data in a database;
select a plurality of second candidate subjects from among the plurality of first candidate subjects by using a second query corresponding to the plurality of first data and a plurality of variable conditions;
select a plurality of third candidate subjects from among the plurality of second candidate subjects based on the displaying of information corresponding to at least a part of second data corresponding to the plurality of second candidate subjects; and
select the clinical trial subjects based on the displaying of information corresponding to at least a part of third data corresponding to each of the plurality of third candidate subjects,
wherein the information corresponding to the at least a part of the second data corresponding to the plurality of second candidate subjects comprises information classified by preset items and displayed,
wherein the selecting of the plurality of third candidate subjects from among the plurality of second candidate subjects comprises:
changing a condition corresponding to at least one item among the preset items based on receiving a user input regarding at least a part of the information classified by the preset items and displayed; and
selecting the plurality of third candidate subjects based on the changed condition;
wherein the information classified by the preset items and displayed includes statistical information for each item of the plurality of second candidate subjects, and
wherein the preset items include age, gender, diagnosis, a physician in charge, a medical department, hospital visit history, medication history, or test information.
